# EUROPEAN PATENT SPECIFICATION

(11) **EP 1 842 572 B1**
(45) Date of publication and mention of the grant of the patent: **30.10.2013**
(21) Application number: 07005569.4
(22) Date of filing: 19.03.2007
(51) Int. Cl.: A61K 8/365, A61K 8/63, A61Q 5/00, A61Q 5/02

(54) **Hair cleansing composition**
Haarwaschmittel
Composition lavante pour les cheveux

(30) Priority: 23.03.2006 JP 2006080782
(43) Date of publication of application: 10.10.2007
(73) Proprietor: KAO CORPORATION, Chuo-ku Tokyo 103-8210 (JP)
(72) Inventor: Sakai, Atsushi, Tokyo 131-8501 (JP); Kasuga, Kennichi, Tokyo 131-8501 (JP); Suzuki, Aya, Tokyo 131-8501 (JP); Kojima, Kaori, Tokyo 131-8501 (JP)
(74) Representative: HOFFMANN EITLE

(56) References cited:
- EP-A- 1 340 487
- WO-A-92/21320
- JP-A- 5 279 228
- JP-A- 7 157 415
- JP-A- 63 022 506
- JP-A- 63 060 909
- JP-A- 2005 187 398
- US-A- 5 785 962
- US-B1- 6 231 843
- TOROK E ET AL: "Skin and soft tissue infections", MEDICINE - U K EDITION, THE MEDICINE PUBLISHING COMPANY, GB, vol. 33, no. 4, 1 April 2005 (2005-04-01), pages 84-88, XP025344368, ISSN: 1357-3039, DOI: 10.1383/MEDC.33.4.84.64357 [retrieved on 2005-04-01]
- 'Pityrosporum folliculitis', [Online] 04 June 2004, Retrieved from the Internet: <URL:http://dermnetnz.org/fungal/pityrospor um-folliculitis.html> [retrieved on 2011-10-21]

## Description

### FIELD OF THE INVENTION

The present invention is related to a hair cleansing composition.

### BACKGROUND OF THE INVENTION

The primary goal of hair shampoo is to remove dirties from hair and scalp to keep them clean. In order to obtain a composition having high-performance in scouring stubborn dirties and high detergency, anionic surfactants, a major cleaning ingredient, have been used in combination of two or more, or used with other suitable surfactants such as amphoteric surfactant and nonioninc surfactant. However, mere use of high detergency surfactants causes a problem that hair becomes coarse on rinsing. Thus, a conditioning-type shampoo which provides hair with easy finger-combing property, suppleness on shampooing and the like has been needed, and cationic polymers or silicones have been used therefor. Although these conditioning agents strongly provide hair conditioning effect, scalp is less conditioned so that excessive amount of sebum is removed from the scalp as the result of the high detergency of the agents. Consequently, the number of people suffering from dry scarp, such as dry feeling, is recently increasing. However, if the amount of surfactants is reduced to protect scalp, the detergency is of course lowered, and dirties remain on the scalp, thereby causing dandruff or itching.

It had been indicated that dandruff, itching or the like is caused by proliferation of microorganisms. To inhibit the dandruff or itching derived from the proliferation of the microorganisms, an antidandruff hair cosmetic composition which includes antimicrobials or bactericides, such as pyridine thionate such as zinc pyrithione, benzalkonium chloride, or octopirox has been used (see, e.g., US Patent No. 3,753,916 and JP-A-58-196300).

Recently, however, due to the increase in frequency of hair washing, dandruff, itching and dryness mainly derived from dryness of scalp has been focused as a major problem for scalp rather than dandruff mainly derived from the proliferation of the microorganisms. Therefore, there has been a need for a hair cleansing composition maintaining high detergency both on hair and scalp, as well as having scalp-care effect and inhibiting dandruff and itching.

### SUMMARY OF THE INVENTION

The present invention provides a hair cleansing composition which contains the following components (A), (B) and (C):
(A) 0.1 to 20 wt.% of a dicarboxylic acid which may have a hydroxy group, a hydroxymonocarboxylic acid, or a salt thereof;
(B) 0.001 to 10 wt.% of an at least one antiphlogistic, wherein the antiphlogistic is at least one anti-inflammatory agent selected from dipotassium glycyrrhizinate, disodium glycyrrhizinate, trisodium glycyrrhizinate, monoammonium glycyrrhizinate, glycerin glycyrrhetate, and stearyl glycyrrhetate;
(C) 0.5 to 60 wt.% of an anionic surfactant,
wherein the composition diluted 20 times by weight with water has a pH of 1 to 5 at 25°C.

### DETAILED DESCRIPTION OF THE INVENTION

The present invention is related to a hair cleansing composition with excellent scalp-care effect, namely it inhibits dryness of the scalp.

The inventors of the present invention have found that a hair cleansing composition with excellent scalp-care effect, namely inhibition of dryness of the scalp, can be provided by preparing a cleansing which includes anionic surfactant as cleaning base, combining it with specific organic acid and antiphlogistics, and adjusting it to certain low pH range.

A hair cleansing composition of the present invention has pH 1 to 5 at 25°C when diluted 20 times by weight with water. In terms of their inhibitory and improving effect on dandruff, itching, pimple and dryness of scalp (hereinafter, referred to as scalp condition-improving effect), preferred pH range is 2 to 4, more preferably 3 to 4.

Examples of the dicarboxylic acid which may have a hydroxy group as component (A), include dicarboxylic acid without having hydroxy group, such as malonic acid, succinic acid, glutaric acid, adipic acid, maleic acid, fumaric acid, phthalic acid and oxalic acid; and hydroxydicarboxylic acid such as malic acid and tartaric acid. Examples of the hydroxymonocarboxylic acid include glycolic acid, lactic acid, hydroxyacrylic acid, oxybutyric acid, and glyceric acid. Among them, lactic acid, malonic acid, maleic acid and malic acid are preferable. Examples of the salt of the organic carboxylic acid include those with alkali metal, alkaline earth metal, ammonia, or organic amine compound.

In the present invention, the component (A) is not only used to adjust pH, but it is essential active ingredient. The component (A) has to be included in the shampoo composition of the invention in an amount of 0.1 to 20 wt.%. Preferably, it is included in an amount of 0.1 to 5 wt.%, more preferably 0.15 to 4 wt.%, and still more preferably 0.2 to 3 wt.%, in order to attain sufficient scalp condition-improving effect.

The antiphlogistics as the component (B) are anti-inflammatory agents selected from dipotassium glycyrrhizinate, disodium glycyrrhizinate, trisodium glycyrrhizinate, monoammonium glycyrrhizinate, glycerin glycyrrhetate, and stearyl glycyrrhetate. The antiphlogistics as the component (B) is included in the present hair cleansing composition in an amount of 0.001 to 10 wt.%, preferably 0.005 to 5 wt.%, and more preferably 0.01 to 1 wt.%.

As the anionic surfactant as the component (C), surfactant containing sulfate group, sulfonate group, or carboxylic group may be used. Examples of the anionic surfactant containing sulfate group, sulfonate group or carboxylic group include alkyl sulfate, polyoxyalkylene alkylether sulfate, polyoxyalkylene alkenylether sulfate, alkyl sulfosuccinate ester salt, polyoxyalkylene alkyl sulfosuccinate ester salt, polyoxyalkylene alkylphenyl ether sulfate, higher fatty acid salt, alkane sulfonate and the like. Among them, polyoxyalkylene alkylether sulfate and alkyl sulfate are preferable. Also preferred is those represented by the following formula (1) or (2):

R¹O-(CH₂CH₂O)ₐSO₃M (1)

R²O-SO₃M (2)

wherein, R¹ represents C10-18 alkyl or alkenyl group, R² represents C10-18 alkyl group, M represents alkali metal, alkaline earth metal, ammonium, alkanolamine or basic amino acid, and a is weight average and represents number 1 to 5.

The anionic surfactants may be used in combination of two or more, and included in the present hair cleansing composition in an amount of 0.5 to 60 wt.%, preferably 1 to 30 wt.%, and more preferably 8 to 20 wt.%, in terms of their foamability, flowability in use, and detergency.

The hair cleansing composition of the invention may further include a conditioning component selected from cationic polymer, cationic surfactant, silicones and oil agent, in order to improve hair condition after drying.

Examples of the cationic polymer includes cationized cellulose derivative, cationic starch, cationized guar gum derivative, homopolymer of diallyl quaternary ammonium salt, diallyl quaternary ammonium salt/acrylamide copolymer, quaternary polyvinylpyrolidone derivative, polyglycol polyamine condensate, vinylimidazolium trichloride/vinylpyrolidone copolymer, hydroxyethylcellulose/dimethyldiallyl ammonium chloride copolymer, vinylpyrolidone/quaternary dimethylaminoethyl methacrylate copolymer, polyvinylpyrolidone/alkylamino acrylate copolymer, polyvinylpyrolidone/alkylaminoacrylate/vinylcaprolactam copolymer, vinylpyrolidone/methacrylamido propyl trimethyl ammonium chloride copolymer, alkylacrylamide/acrylate/alkylaminoalkylacrylamide/polyethylene glycol methacrylate copolymer, adipic acid/dimethylamino hydroxypropylethylenetriamine copolymer (Cartaretine, Sandoz Inc., USA), and cationic polymers disclosed in JP-A-53-139734, and JP-A-60-36407. Among them, cationized cellulose derivative, cationized guar gum derivative, and diallyl quaternary ammonium salt/acrylamide copolymer are preferable. The cationic polymer may be used in combination, and contained in the present-hair cleansing composition preferably in an amount of 0.02 to 5 wt.%, more preferably 0.05 to 1 wt.%, and still more preferably 0.1 to 0.7 wt.%, in terms of improvement in their foamability on washing, as well as in manageability and touching feel of hair after drying.

Examples of the cationic surfactant include alkyl trimethyl ammonium salt such as lauryl trimethyl ammonium chloride, cetyl trimethyl ammonium chloride, cetyl trimethyl ammonium bromide, stearyl trimethyl ammonium chloride, stearyl trimethyl ammonium bromide and lauryl trimethyl ammonium bromide; and dialkyl dimethyl ammonium salt such as dicetyl dimethyl ammonium chloride, distearyl dimethyl ammonium chloride and dicocoyl dimethyl ammonium chloride. Another cationic surfactant which can be used includes myristyl dimethylbenzyl ammonium chloride, stearyl dimethylbenzyl ammonium chloride, lanolin fatty acid aminoethyl trimethyl ammonium methyl sulfate, lanolin fatty acid aminopropylethyl dimethyl ammonium methyl sulfate, lanolin fatty acid aminopropylethyl dimethyl ammonium ethyl sulfate, lanolin fatty acid aminopropyl triethyl ammonium ethyl sulfate, lanolin fatty acid aminoethyl triethyl ammonium ethyl sulfate, isostearic acid aminopropylethyl dimethyl ammonium ethyl sulfate, isononanoic acid aminopropylethyl dimethyl ammonium ethyl sulfate, stearyl amidopropyl dimethylamine (and salt thereof), stearyl amidoethyl diethylamine (and salt thereof), isoalkanoic acid (C14 to C20) aminopropylethyl dimethyl ammonium ethyl sulfate, isoalkanoic acid (C18 to C22) aminopropylethyl dimethyl ammonium ethyl sulfate, and alkyl trimethyl ammonium saccharine. The cationic surfactant may be used in combination, and included in the present hair cleansing composition preferably in an amount of 0.01 to 10 wt.%, more preferably 0.05 to 6 wt.%, still more preferably 0.3 to 3 wt.%, and still more preferably 0.5 to 2 wt.%, in terms of hair suppleness during from shampooing to rinsing.

Examples of the silicones include those described below. (Silicones-1) Dimethylpolysiloxane

R³ (CH₃) ₂SiO- [(CH₃) ₂SiO]_{b}-Si (CH₃)₂R³

wherein R³ represents methyl or hydroxy group, and b represents a number of 3 to 20000.

Examples of such silicones include BY11-026, BY22-19 (Dow Corning Toray, Co., Ltd.), and FZ-3125 (Nippon Unicar Co., Ltd.). Dimethylpolysiloxane high polymer can be used as a solution or dispersion in liquid oil (e.g., oil of dimethylpolysiloxane low polymer, liquid silicone oil such as circular silicone, or liquid hydrocarbon oil such as isoparaffin).

### (Silicones-2) Amino modified silicone

Various amino modified silicones can be used, but preferred is those described in CTFA Dictionary (Cosmetic Ingredient Dictionary, the 3rd edition, USA) under the name of Amodimethicone, and with their averaged molecular weight of about 3000 to 100000. This amino modified silicone is preferably used as aqueous emulsion. Example of those commercially-available includes SM8704C (Dow Corning Toray, Co., Ltd.), and DC 929 (Dow Corning Corporation).

Another example of the amino modified silicone includes a compound represented by the following formula: 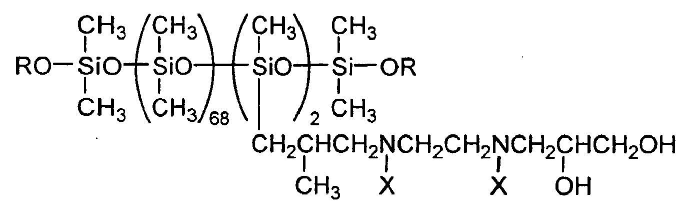 wherein R represents C13-15 linear or branched alkyl group, 75% of X represents group -CH₂CH(OH)CH₂OH, and remaining 25% represents hydrogen atom.

Example of those commercially-available includes "8500 Conditioning Agent" (Dow Corning Corporation, CAS No. 237753-63-8).

Preferred examples of the amino modified polysiloxane-polyoxyalkylene block copolymer include those represented by the following formula: 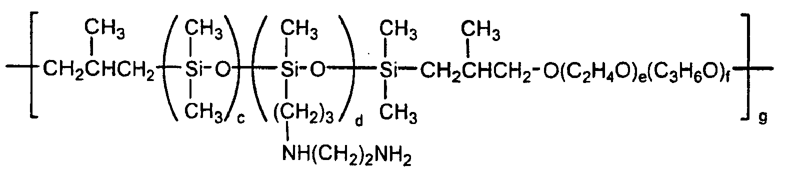 wherein c represents a number of 2 or more, d represents a number of 1 or more, e represents a number of 4 or more, f represents a number of 0 to 30, and g represents a number of 2 or nore.

Preferably, in the above formula, c represents a number of 2 to 1,000, d represents a number of 1 to 50, e represents a number of 4 to 200, and g represents a number of 2 to 100. - O(C₂H₄O)ₑ(C₃H₆O)_{f}- may be block copolymer or random copolymer. Examples of those commercially-available include FZ-3789 and silicone SS-3588 (Dow Corning Toray, Co., Ltd.).

### (Silicones-3) Other silicones

Also included is polyether modified silicone, methylphenyl polysiloxane, fatty acid modified silicone, alcohol modified silicone, alkoxy modified silicone, epoxy modified silicone, fluorine modified silicone, cyclic silicone, and alkyl modified silicone.

These silicones may be used in combination of two or more, and included in the present hair cleansing composition preferably in an amount of 0.1 to 7 wt.%, more preferably 0.2 to 6 wt.%, and still more preferably 0.5 to 5 wt.% , in terms of smoothness, ease in finger-combing, and sleekness of the hair.

The oil agent is referred to as oily substance except for silicones, and includes hydrocarbons such as squalene, squalan, liquid paraffin, liquid isoparaffin and cycloparaffin; glycerides such as castor oil, cacao oil, mink oil, avocado oil and olive oil; waxes such as bees wax, whale wax, lanolin and carnauba wax; alcohols such as cetyl alcohol, oleyl alcohol, stearyl alcohol, isostearyl alcohol, and 2-octyl dodecanol; esters such as isopropyl palmitate, isopropyl myristate, octyl dodecyl myristate, hexyl laurate, cetyl lactate, propylene glycol monostearate, oleyl oleate, hexadecyl 2-ethylhexanoate, isononyl isononanoate and tridecyl isononanoate; higher fatty acids such as capric acid, lauric acid, myristic acid, palmitic acid, stearic acid, behenic acid, oleic acid, palm oil fatty acid, isostearic acid and isopalmitic acid; isotearyl glyceryl ether, and polyoxypropylene butylether. Among them, esters are preferred. Hexadecyl 2-ethylhexanoate, isononyl isononanoate, and isopropyl palmitate are more preferred. These oil agents may be used in combination of two or more, and included in the present hair cleansing composition preferably in an amount of 0.2 to 2 wt.%, more preferably 0.3 to 1.8 wt.%, and still more preferably 0.5 to 1.5 wt.%.

The above conditioning components may be used in combination of two or more, and included in the present hair cleansing composition preferably in an amount of 0.05 to 10 wt.%, more preferably 0.1 to 5 wt.%, still more preferably 0.3 to 2 wt.%, in terms of lubricity of foam, and smoothness of hair during from shampooing to rinsing.

The hair cleansing composition of the present invention may include a surfactant selected from nonionic surfactants and amphoteric surfactants, in order to further improving the performance of foam.

The nonionic surfactants include polyoxyalkylene sorbitan fatty acid esters, polyoxyalkylene sorbit fatty acid esters, polyoxyalkylene glycerine fatty acid esters, polyoxyalkylene fatty acid esters, alkyl glyceryl ethers, polyoxyalkylene alkylethers, polyoxyalkylene alkyl phenyl ethers, polyoxyalkylene (hydrogenated) castor oils, sucrose fatty acid esters, polyglycerol alkyl ethers, polyglycerol fatty acid esters, fatty acid alkanol amide, and alkyl glycosides. Among them, glycerol monoalkylether, alkyl glycosides, polyoxyalkylene (C₈ to C₂₀) fatty acid ester, polyoxyethylene sorbitan fatty acid ester, polyoxy ethylene hydrogenated castor oil, and fatty acid alkanol amide are preferable. The preferred fatty acid alkanol amides include those having C8-18 acyl group, preferably C10-16 acyl group. The fatty acid alkanol amide may be monoalkanol amide or dialkanol amide, and preferably having C2-3 hydroxyalkyl group, including diethanolamide oleate, palm kernel oil fatty acid diethanolamide, palm oil fatty acid diethanolamide, diethanolamide laurate, polyoxyethylene palm oil fatty acid monoethanolamide, palm oil fatty acid monoethanolamide, isopropanolamide laurate, and monoethanolamide laurate.

The amphoteric surfactant includes betainic surfactant. Among them, betainic surfactant such as alkyl dimethylamino betaine acetate or fatty acid amidopropyl betaine is more preferable, and fatty acid amidopropyl betaine is more preferable. Fatty acid amidopropyl betaine having C8-18 acyl group is preferable, those having C10-16 acyl group is more preferable. Specifically preferred are lauramidopropyl betaine, palm kernel oil fatty acid amidopropyl betaine, and palm oil fatty acid amidopropyl betaine.

In addition to the components described above, the hair cleansing composition of the present invention may optionally contain other components commonly used for hair cleansing compositions, depending on its application. Such components include chelating agent; moisturizing agent such as sorbitol and panthenol; colorant such as dyes and pigment; viscosity modifier such as hydroxyethylcellulose, methylcellulose, polyethyleneglycol, polyvinyl alcohol and clay mineral; pH adjuster such as potassium hydroxide, sodium hydroxide and citric acid; plant extracts; pearling agent; perfume; UV absorber; antioxidant; and components described in ENCYCLOPEDIA OF SHAMPOO INGREDIENTS (MICELLE PRESS).

The hair cleansing composition of the present invention has excellent inhibiting effect on dandruff, itching, pimple and dryness of scalp, thus being useful for antidandruff shampoo.

### EXAMPLES

The pH values indicated in the following Examples and comparative examples were obtained at 25°C after diluting 20 times by weight with water.

### Examples 1 to 3 and comparative examples 1 to 4

Shampoo compositions shown in Table 1 were prepared and evaluated.

### (Evaluation of scalp condition-improving effect)

A group of forty subjects (twenty men and twenty women) was selected from ordinary men and women in their 20's to 40's, wherein dandruff and pimple were visually found in their scalp. The subjects used control shampoo for a month, and then used test shampoo for about 6 weeks to evaluate change in scalp condition visually.

Dandruff condition was evaluated by sorting the scalp into 12 areas, then classifying each area into level 0 to level 6 (7 levels), and summing up the level scores to sort each scalp into level 0 to level 72. Level 10 or more was defined as dandruff group. Pimple group was defined as pimple condition in which there were 3 pimples or more in whole scalp area. Scalp was classified into normal group if dandruff and pimple conditions did not fall into the criteria for dandruff and pimple groups described above.

Evaluation of dandruff and pimple was conducted by expert researchers under double-blind condition. The results were ranked according to the averaged change in dandruff level and the number of pimple before and after using the shampoo. Table 1 shows the results.

Evaluation of itching and dryness was conducted by ranking the averaged score (rounded to the tenth place) obtained from questionnaire in which the subjects wrote down their answer with five-grade evaluation (5 - improved; 4 - slightly improved; 3 - not changed; 2 - slightly worsened; and 1 - worsened). Table 1 shows the results. The formulation of control shampoo is described below.

| Control shampoo | |
|---|---|
| | (wt.%) |
| Sodium polyoxyethylene(2) lauryl ether sulfate | 13.5 |
| Palm oil fatty acid monoethanolamide | 0.5 |
| Polyoxyethylene(16)lauryl ether | 2 |
| Lauramidopropyl betaine | 1.5 |
| Cationized cellulose | 0.5 |
| pH Adjuster | |
| (citric acid, 48 wt.% aqueous sodium hydroxide) | to pH 5.5^{*} |
| Purified water | Balance |

| | |
|---|---|
| ^{*}: pH at 25°C obtained by diluting 20 times by weight with water | |

### <Dandruff rank>

A (Effective): Improvement (≥ 10 levels) in averaged dandruff level before and after the use
B (Slightly effective): Improvement (≥ 5 levels) in averaged dandruff level before and after the use
C (Not changed): Less than ±5 level change in averaged dandruff level before and after the use
D (Worsened): Worsening (≥ 5 levels) in averaged dandruff level before and after the use

### <Pimple rank>

A (Effective): Decrease (≥ 5) in averaged pimple number before
and after the use
B (Slightly effective): Decrease (≥ 3) in averaged pimple number before and after the use
C (Not changed): Less than ±3 of change in averaged pimple number before and after the use
D (Worsened): Increase (≥ 3) in averaged pimple number before and after the use

### <Itching rank>

A (Effective): Averaged ranking score of ≥ 3.5
B (Slightly effective): Averaged ranking score of 3.2 to 3.4
C (Not changed): Averaged ranking score of 2.8 to 3.1
D (Worsened): Averaged ranking score of ≤ 2.7

### <Dryness rank>

A (Effective): Averaged ranking score of ≥ 3.5
B (Slightly effective): Averaged ranking score of 3.2 to 3.4
C (Not changed): Averaged ranking score of 2.8 to 3.1
D (Worsened): Averaged ranking score of ≤ 2.7

**Table 1**

| Components (wt.%) | | Examples | | | Comparative examples | | | |
|---|---|---|---|---|---|---|---|---|
| | | 1 | 2 | 3 | 1 | 2 | 3 | 4 |
| Sodium polyoxyethylene(2) lauryl ether sulfate | | 13.5 | 13.5 | 13.5 | 13.5 | 13.5 | 13.5 | 13.5 |
| Palm oil fatty acid monoethanolamide | | 0.5 | 0.5 | 0.5 | 0.5 | 0.5 | 0.5 | 0.5 |
| Polyoxyethylene(16)lauryl ether | | 2 | 2 | 2 | 2 | 2 | 2 | 2 |
| Lauramidopropyl betaine | | 1.5 | 1.5 | 1.5 | 1.5 | 1.5 | 1.5 | 1.5 |
| Malic acid | | 0.75 | - | 0.75 | 0.75 | - | 0.75 | - |
| Lactic acid | | - | 0.75 | - | - | - | - | - |
| Zinc pyrithione | | - | - | - | - | - | - | 0.8 |
| Dipotassium glycyrrhizinate | | 0.1 | 0.1 | - | - | 0.1 | 0.1 | - |
| Stearyl glycyrrhetate | | - | - | 0.1 | - | - | - | - |
| Cationized cellulose | | 0.5 | 0.5 | 0.5 | 0.5 | 0.5 | 0.5 | 0.5 |
| Citric acid | | - | - | - | - | 0.75 | - | 0.1 |
| 48 wt.% Aqueous sodium hydroxide | | q.s.^{*} | q.s.^{*} | q.s.^{*} | q.s.^{*} | q.s.^{*} | q.s.^{*} | q.s.^{*} |
| Pure water | | Balance | Balance | Balance | Balance | Balance | Balance | Balance |
| pH (diluted 20 times by weight with water, at 25°C) | | 3.7 | 3.7 | 3.7 | 3.7 | 3.7 | 5.5 | 5.5 |
| Rank | Dandruff | A | A | A | B | B | B | A |
| | Itching | A | A | A | C | B | B | A |
| | Pimple | A | A | A | B | C | C | C |
| | Dryness | A | A | A | C | C | C | C |

| | | | | | | | | |
|---|---|---|---|---|---|---|---|---|
| ^{*}: volume to adjust pH | | | | | | | | |

Shampoos of Example 1 to 3 exhibited excellent inhibiting effect on dandruff, itching, pimple and dryness of scalp. In contrast, comparative example 1 without containing antiphlogistics exhibited less improvement effect, particularly on itching and dryness. For comparative example 2 without organic acid of component (A) but with citric acid, improvement effect was not found, particularly on pimple or dryness. For comparative example 3 with organic acid of component (A) and having higher pH, improvement effect on dandruff, itching, pimple and dryness was not found. For comparative example 4 with zinc pyrithione which is commonly utilized antimicrobial used at pH5 or more, inhibitory effect on pimple or dryness was weak.

### Example 4 Transparent shampoo

| | (wt.%) |
|---|---|
| Dipotassium glycyrrhizinate | 0.1 |
| Sodium polyoxyethylene(2) lauryl ether sulfate | 13.5 |
| Palm oil fatty acid monoethanolamide | 0.4 |
| Lauramidopropyl betaine | 1.5 |
| Polyoxyethylene(16)lauryl ether | 2.0 |
| Cationized cellulose | 0.5 |
| Malic acid | 0.8 |
| 48 wt.% Aqueous sodium hydroxide | q.s. |
| Perfume | q.s. |
| Purified water | Balance |

This shampoo (pH = 3.7) showed excellent inhibiting effect on dandruff, itching, pimple and dryness of scalp.

### Example 5 Pearl-like shampoo

| | (wt.%) |
|---|---|
| Dipotassium glycyrrhizinate | 0.1 |
| Sodium polyoxyethylene(2) lauryl ether sulfate | 13.5 |
| Palm oil fatty acid monoethanolamide | 0.5 |
| Lauramidopropyl betaine | 1.5 |
| Polyoxyethylene(16)lauryl ether | 2.0 |
| Ethyleneglycol distearate | 2.0 |
| Cationized cellulose | 0.5 |
| Methylpolysiloxane high polymer | |
| (degree of polymerization 1000 to 2700) | 1.0 |
| Methylpolysiloxane | |
| (degree of polymerization 50 to 300) | 1.4 |
| Malic acid | 0.8 |
| Sodium chloride | 0.2 |
| 48 wt.% Aqueous sodium hydroxide | q.s. |
| Perfume | q.s. |
| Purified water | Balance |

This shampoo (pH = 3.7) showed excellent inhibiting effect on dandruff, itching, pimple and dryness of scalp.

## Claims

1. A hair cleansing composition for use in a method of inhibiting dryness of the scalp, the composition comprising the following components (A), (B) and (C):
(A) 0.1 to 20 wt.% of an organic dicarboxylic acid which may have a hydroxy group, hydroxymonocarboxylic acid, or a salt thereof;
(B) 0.001 to 10 wt.% of an at least one antiphlogistic, wherein the antiphlogistic is at least one anti-inflammatory agent selected from dipotassium glycyrrhizinate, disodium glycyrrhizinate, trisodium glycyrrhizinate, monoammonium glycyrrhizinate, glycerin glycyrrhetate and stearyl glycyrrhetate;
(C) 0.5 to 60 wt.% of an anionic surfactant,
wherein the composition diluted 20 times by weight with water has a pH of 1 to 5 at 25 °C.

2. The hair cleansing composition for use in a method of inhibiting dryness of the scalp according to claim 1, wherein the at least one anti-inflammatory agent is selected from dipotassium glycyrrhizinate and stearyl glycyrrhetate.

3. The hair cleansing composition for use in a method of inhibiting dryness of the scalp according to claim 2, wherein the at least one anti-inflammatory agent is dipotassium glycyrrhizinate.

4. The hair cleansing composition for use in a method of inhibiting dryness of the scalp according to claim 2, wherein the at least one anti-inflammatory agent is stearyl glycyrrhetate.

5. The hair cleansing composition for use in a method of inhibiting dryness of the scalp according to any one of claims 1 to 4, wherein the method comprises applying the hair cleansing composition to the scalp followed by washing off the composition.

## Patentansprüche

1. Haarreinigungszusammensetzung zur Verwendung in einem Verfahren zur Inhibierung der Trockenheit der Kopfhaut, wobei die Zusammensetzung die nachstehenden Komponenten (A), (B) und (C) umfasst:
(A) 0,1 bis 20 Gew.% einer organischen Dicarbonsäure, die eine Hydroxygruppe haben kann, einer Hydroxymonocarbonsäure oder einem Salz davon;
(B) 0,001 bis 10 Gew.% mindestens eines Antiphlogistikums, wobei das Antiphlogistikum mindestens ein entzündungshemmendes Mittel, ausgewählt aus Dikaliumglycyrrhizinat, Dinatriumglycyrrhizinat, Trinatriumglycyrrhizinat, Monoammoniumglycyrrhizinat, Glyceringlycyrrhetat und Stearylglycyrrhetat, ist;
(C) 0,5 bis 60 Gew.% eines anionischen Tensids, wobei die mit Wasser auf das 20-fache ihres Gewichts verdünnte Zusammensetzung einen pH-Wert von 1 bis 5 bei 25°C aufweist.

2. Haarreinigungszusammensetzung zur Verwendung in einem Verfahren zur Inhibierung der Trockenheit der Kopfhaut gemäss Anspruch 1, wobei das mindestens eine entzündungshemmende Mittel aus Dikaliumglycyrrhizinat und Stearylglycyrrhetat ausgewählt ist.

3. Haarreinigungszusammensetzung zur Verwendung in einem Verfahren zur Inhibierung der Trockenheit der Kopfhaut gemäss Anspruch 2, wobei das mindestens eine entzündungshemmende Mittel Dikaliumglycyrrhizinat ist.

4. Haarreinigungszusammensetzung zur Verwendung in einem Verfahren zur Inhibierung der Trockenheit der Kopfhaut gemäss Anspruch 2, wobei das mindestens eine entzündungshemmende Mittel Stearylglycyrrhetat ist.

5. Haarreinigungszusammensetzung zur Verwendung in einem Verfahren zur Inhibierung der Trockenheit der Kopfhaut gemäss irgendeinem der Ansprüche 1 bis 4, wobei das Verfahren das Aufbringen der Haarreinigungszusammensetzung auf die Kopfhaut, gefolgt von dem Auswaschen der Zusammensetzung, umfasst.

## Revendications

1. Composition de nettoyage de cheveux à utiliser dans un procédé d'inhibition de la sécheresse du cuir chevelu, la composition comprenant les composants suivants (A), (B) et (C) :
(A) de 0,1 à 20% en poids d'un acide dicarboxylique organique qui peut avoir un groupe hydroxy, d'un acide hydroxymonocarboxylique, ou d'un sel de ceux-ci ;
(B) de 0,001 à 10% en poids d'au moins un antiphlogistique, où l'antiphlogistique est au moins un agent anti-inflammatoire choisi parmi le glycyrrhizinate dipotassique, le glycyrrhizinate disodique, le glycyrrhizinate trisodique, le glycyrrhizinate monoammonique, la glycérine glycyrrhetate et le stearyl glycyrrhetate ;
(C) de 0,5 à 60% en poids d'un tensioactif anionique,
où la composition diluée 20 fois en poids avec de l'eau a un pH allant de 1 à 5 à 25°C.

2. Composition de nettoyage de cheveux à utiliser dans un procédé d'inhibition de la sécheresse du cuir chevelu selon la revendication 1, dans laquelle l'au moins un agent anti-inflammatoire est choisi parmi le glycyrrhizinate dipotassique et le stearyl glycyrrhetate.

3. Composition de nettoyage de cheveux à utiliser dans un procédé d'inhibition de la sécheresse du cuir chevelu selon la revendication 2, dans laquelle l'au moins un agent anti-inflammatoire est le glycyrrhizinate dipotassique.

4. Composition de nettoyage de cheveux à utiliser dans un procédé d'inhibition de la sécheresse du cuir chevelu selon la revendication 2, dans laquelle l'au moins un agent anti-inflammatoire est le stearyl glycyrrhetate.

5. Composition de nettoyage de cheveux à utiliser dans un procédé d'inhibition de la sécheresse du cuir chevelu selon l'une quelconque des revendications 1 à 4, dans laquelle le procédé comprend l'application de la composition de nettoyage de cheveux sur le cuir chevelu, suivie d'un lavage de la composition.
